(19) Europäisches Patentamt
European Patent Office
Office européen des brevets

(11) Veröffentlichungsnummer: **0 389 961 A1**

(12) **EUROPÄISCHE PATENTANMELDUNG**

(21) Anmeldenummer: **90105404.9**

(22) Anmeldetag: **22.03.90**

(51) Int. Cl.5: **C07C 39/367, C07C 43/29, C07C 205/38, C07C 65/24, C07C 235/42, C07C 217/80, C07C 217/82, C07C 41/09, C07C 51/60, C07C 201/12, C07C 51/215, //C07C231/02, C07C213/02,C08G69/26**

(30) Priorität: **25.03.89 DE 3909881**

(43) Veröffentlichungstag der Anmeldung: **03.10.90 Patentblatt 90/40**

(84) Benannte Vertragsstaaten: **BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Lau, Jürgen, Dr.**
**Breitenweisenstrasse 24**
**D-8751 Haibach(DE)**
Erfinder: **Siegemund, Günter, Dr.**
**Frankfurter Strasse 21**
**D-6238 Hofheim am Taunus(DE)**
Erfinder: **Röhrscheid, Freimund, Dr.**
**Amselweg 24**
**D-6233 Kelkheim/Taunus(DE)**

(54) **Hexafluorisopropylgruppenhaltige Monomere, Verfahren zu ihrer Herstellung und ihre Verwendung.**

(57) Verbindungen der Formel I

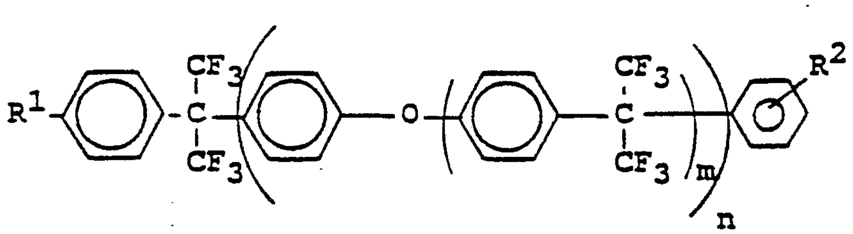

(I)

lassen sich durch unterschiedliche Verfahrensschritte aus 2-(4-Methylphenyl)-2-hexafluorisopropanol herstellen.

Hexafluorisopropylhaltige Monomere sind bei der Herstellung von linearen Polycarbonsäureamiden und -imiden wichtige Ausgangsverbindungen.

EP 0 389 961 A1

## Hexafluorisopropylgruppenhaltige Monomere, Verfahren zu ihrer Herstellung und ihre Verwendung

Die Erfindung betrifft hexafluorisopropylgruppenhaltige Monomere, Verfahren zu ihrer Herstellung und ihre Verwendung.

Teilfluorierte aromatische Diamine sind wertvolle Monomere für hochtemperaturbeständige Polykondensate wie Polyamide und Polyimide.

Es ist bekannt, daß aus teilfluorierten aromatischen Diaminen hochtemperaturbeständige Polymere hergestellt werden können. 2,2-Bis[4-(4-aminophenoxy)phenyl]hexafluorpropan, das zwei Etherbrücken und eine Hexafluorisopropylidenbrücke enthält, wird zur Herstellung von Polyimiden mit günstigen chemischen und thermischen Eigenschaften eingesetzt (vgl. US 4,111,906). Die Einführung von Etherbrücken in Polyimiden vergrößert dabei die Flexibilität der Hauptkette, erniedrigt die Glasübergangstemperatur und verbessert die Verarbeitungseigenschaften (vgl. J. Polym. Sci. 74, 93 (1986)).

Die Anzahl der verfügbaren aromatischen Diamine, welche über Etherbrücken und Hexafluorisopropylidenbrücken verfügen, ist allerdings begrenzt.

Es bestand daher die Aufgabe, neue teilfluorierte aromatische Diaminoverbindungen mit Etherbrücken für die Herstellung von Polyimiden mit günstigen thermischen und chemischen Eigenschaften herzustellen.

Die Aufgabe wird gelöst durch die Bereitstellung von hexafluorisopropylgruppenhaltigen Monomeren und damit auch teilfluorierten aromatischen Diaminoverbindungen, die sich, ausgehend von 2-(4-Methylphenyl)-2-hexafluorisopropanol, über mehrere Verfahrensschritte herstellen lassen.

Die Erfindung betrifft Verbindungen der Formel I

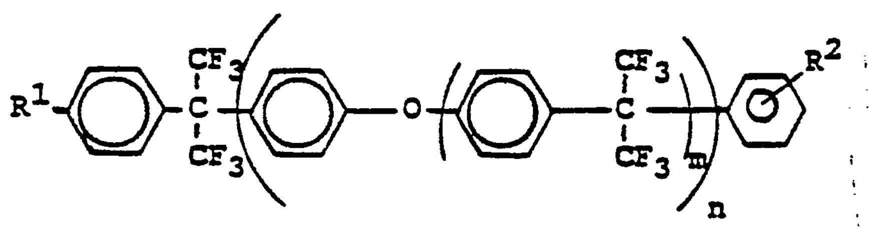 (I),

worin
m und n null oder 1 bedeuten, und wenn
m = null und n = null sind, dann sind
$R^1$ $-CH_3$ und
$R^2$ -OH in para-Stellung, wenn
m = null und n = 1 sind, dann sind
$R^1$ $-CH_3$, -COOH, -COCl, $-CONH_2$ und $-NH_2$ und
$R^2$ $-CH_3$, -COOH, -COCl, $-CONH_2$, $-NH_2$ und $-NO_2$ in meta- oder para-Stellung und wenn
m = 1 und n = 1 sind, dann sind
$R^1$ -COCl, $-CONH_2$, $-NH_2$ und
$R^2$ -COCl, $-CONH_2$, und $-NH_2$.

Die Erfindung betrifft weiterhin Verfahren zur Herstellung einer Verbindung der Formel I mit m = null und n = null gemäß Anspruch 1, dadurch gekennzeichnet, daß 2-(4-Methylphenyl)-2-hexafluorisopropanol mit Phenol in Gegenwart von wasserfreier Flußsäure zu 2-(4-Hydroxyphenyl)-2-(4-methylphenyl)-hexafluorpropan umgesetzt wird.

Die Erfindung betrifft auch Verfahren zur Herstellung von Verbindungen der Formel I mit m = null und n = 1 gemäß Anspruch 1, dadurch gekennzeichnet, daß

1) 2-(4-Hydroxyphenyl)-2-(4-methylphenyl)hexafluorpropan mit einer aromatischen halogenhaltigen Nitroverbindung oder Dinitroverbindung, oder 2-(4-Methylphenyl)-2-hexafluorisopropanol mit 4-Methyldiphenylether in Gegenwart von wasserfreier Flußsäure zu 2-(4-Methylphenyl)-2-[4-(4-methylphenoxy)phenyl]-hexafluorpropan umgesetzt wird,

2) eine in 1) gebildete Verbindung zum Carbonsäurederivat oxidiert wird,

3) das Carbonsäurederivat mit Thionylchlorid zum Säurechlorid,

4) das Säurechlorid mit Ammoniaklösung zum Amid,

5) das Amid mit gelöstem Natriumhydroxid und NaOCl zum Amin umgesetzt und

6) die Nitrogruppe in der nach Ausführung von 5) vorliegenden Aminonitroverbindung katalytisch zum

Amin reduziert wird.

Die Erfindung betrifft außerdem Verfahren zur Herstellung von Verbindungen der Formel I mit m = 1 und n = 1 gemäß Anspruch 1, dadurch gekennzeichnet, daß

1) 4,4′-Bis[2-(4-carboxyphenyl)hexafluorisopropyl]diphenylether mit Thionylchlorid zum Säurechlorid,

2) das Säurechlorid mit Ammoniaklösung zum Amid,

3) das Amid mit gelöstem Natriumhydroxid und NaOCl zum Amin umgesetzt werden.

Die Erfindung betrifft ebenso die Verwendung der Verbindungen gemäß Anspruch 1 zur Herstellung von linearen, Polycarbonsäureamiden und -imiden.

Die Herstellung des 12F-Diamins, 4,4′-Bis[2-(4-aminophenyl)hexafluorisopropyl]diphenylether, erfolgt ausgehend von der 12F-Dicarbonsäure, 4,4′-Bis[2-(4-carboxyphenyl)hexafluorisopropyl]diphenylether über 3 Reaktionsschritte, wobei als Zwischenprodukte die entsprechenden 12-Dicarbonsäurechloride und -amide auftreten.

Die Herstellung des 6F-Diamins, 2-(4-Aminophenyl)-2-[4-(4-aminophenoxy)phenyl]hexafluorpropan, wird nach zwei alternativen Verfahren durchgeführt. Im ersten Verfahren wird ausgehend von 4-Methyldiphenylether und 2-(4-Methylphenyl)-2-hexafluorisopropanol in Gegenwart von wasserfreier Flußsäure die 6F-Dimethylverbindung, 2-(4-Methylphenyl)-2-[4-(4-methylphenoxy)phenyl]hexafluorpropan, hergestellt, diese wird mittels katalytischer Luftoxidation zunächst in die 6F-Dicarbonsäure übergeführt, aus der über das entsprechende 6F-Dicarbonsäurechlorid und -amid letztlich das 2-(4-Aminophenyl)-2-[4-(4-aminophenoxy)phenyl]-hexafluorpropan entsteht.

Im zweiten Verfahren wird ausgehend von Phenol, und 2-(4-Methylphenyl)-2-hexafluorisopropanol in Gegenwart von wasserfreier Flußsäure zunächst 2-(4-Hydroxyphenyl)-2-(4-methylphenyl)hexafluorpropan hergestellt. Letzteres wird mit einem 4-Halogennitrobenzol zu 2-(4-Methylphenyl)-2-[4-(4-nitrophenoxy)-phenyl]hexafluorpropan umgesetzt. Die Methylverbindung wird anschließend oxidiert und über das Carbonsäurechlorid und Carbonsäureamid in 2-(4-Aminophenyl)-2-[4-(4-nitrophenoxy)phenyl]hexafluorpropan übergeführt, aus welchem durch Reduktion der Nitrogruppe 2-(4-Aminophenyl)-2-[4-(4-aminophenoxy)phenyl]-hexafluor propan hergestellt wird.

Analog zum zweiten Verfahren wird ausgehend von 2-(4-Hydroxyphenyl)-2-(4-methylphenyl)-hexafluorpropan und 1,3-Dinitrobenzol ein weiteres 6F-Diamin, 2-(4-Aminophenyl)-2-[4-(3-aminophenoxy)-phenyl]hexafluorpropan, hergestellt.

Die erfindungsgemäßen Mono- und Dimethylverbindungen werden durch Umsetzung von 2-(4-Methylphenyl)-2-hexafluorisopropanol mit Phenol oder 4-Methyldiphenylether in Gegenwart von wasserfreier Flußsäure hergestellt.

Die Ausgangsverbindung 2-(4-Methylphenyl)-2-hexafluorisopropanol ist bekannt und in J. Org. Chem. 998-1001, 30 (1965) beschrieben.

Die Reaktionstemperatur bei dem Kondensationsverfahren liegt zwischen 80 und 180 °C, vorzugsweise zwischen 100 und 170 °C. Die Reaktionszeiten betragen 24 bis 90, vorzugsweise 65 bis 90 Stunden. Das Molverhältnis der eingesetzten Reaktionspartner beträgt in beiden Fällen 1 : 5, vorzugsweise 1 : 1,5 bis 2,5, wobei die nicht fluorhaltigen Reaktionspartner stets die Überschußkomponenten sind. Der Anteil an Fluorwasserstoff wird auf die fluorhaltige Ausgangsverbindung bezogen und liegt bei einem Molverhältnis von 1 : 7 bis 25, vorzugsweise 1 : 9 bis 20. Zur Aufarbeitung des Reaktionsguts wird der Fluorwasserstoff nach Beendigung der Reaktion bei ca. 80 °C aus dem Reaktor abgelassen.

Die erfindungsgemäßen Methylverbindungen werden nach den üblichen stöchiometrischen Methoden mit z. B. Kaliumpermanganat, Chromsäure/Eisessig, Dichromat/Schwefelsäure oder bevorzugt katalytisch mit molekularem Sauerstoff in Gegenwart einer Katalysatorkombination aus den Ionen von Kobalt, Mangan und Brom zu Carbonsäuren oxidiert, wobei in einem sauren Medium gearbeitet wird, das zu mindestens 40 Gew.-% aus Essigsäure oder Propionsäure oder deren Gemischen besteht. Essigsäure ist wegen ihrer größeren Beständigkeit gegen einen oxidativen Abbau vorzuziehen.

Bromidionen sind für den vollständigen Ablauf der Oxidation unbedingt notwendig. Kobalt- und Manganionen werden im Verhältnis von 3 : 1 bis 1 : 3, vorzugsweise 1 : 1 eingesetzt. Die Summe der Konzentrationen der beiden Elemente Kobalt und Mangan beträgt 0,01 bis 0,2, vorzugsweise 0,02 bis 0,12 und insbesondere 0,04 bis 0,08 g/Atom/kg Gesamtmasse. Das Verhältnis der Summe von Kobalt und Mangan zu Brom ist 1 : 0,01 bis 2, vorzugsweise 1 : 0,025 bis 1 und insbesondere 1 : 0,05 bis 0,2. Es ist auch möglich, zusätzlich zu den beiden Metallionen des Katalysators noch Cerionen einzusetzen. Diese katalysieren die Oxidation der unvollständig oxidierten Zwischenstufen. Ihre Anwesenheit erhöhen die Reinheit und die Ausbeute der teilfluorierten Monocarbonsäure. Die Cerionen werden dem Katalysator im Verhältnis der Summe der Kobalt- und Manganionen zu Cerionen wie 1 : 0,02 bis 2, vorzugsweise 1 : 0,05 bis 1 und insbesondere 1 : 0,2 bis 0,6 zugefügt. Vorzugsweise werden die Metallionen in Form ihrer Acetate eingesetzt.

Brom wird in Form von Bromiden, z. B. der Alkalimetalle, einschließlich des Ammoniumbromids, und denen der Metalle Kobalt, Mangan und Cer oder als Lösung von Bromwasserstoff in Wasser oder Eisessig eingesetzt werden. Es können auch bromhaltige organische Verbindungen verwendet werden, die während der Oxidation zerfallen und Bromionen freisetzen, beispielsweise Tetrabrommethan.

Die Oxidation wird bei Temperaturen von 120 bis 220 °C, vorzugsweise 140 bis 190 °C und insbesondere 155 bis 180 °C durchgeführt. Der Druck im Reaktor liegt zwischen 5 und 40 bar, vorzugsweise zwischen 10 bis 30 bar und insbesondere zwischen 14 bis 20 bar.

Für die Oxidation ist es günstig, daß die benötigte Luft nahe am Boden des Reaktors in die Flüssigphase eingeleitet wird und durch starkes Rühren oder durch spezielle Düsen in der Flüssigphase fein verteilt wird.

Als Ausgangsmaterialien für die erfindungsgemäßen Nitroverbindungen dienen beispielsweise 2-(4-Hydroxyphenyl)-2-(4-methylphenyl)hexafluorpropan und 2-(4-Carboxyphenyl)-2-(4-hydroxyphenyl)-hexafluorpropan, welche mit aromatischen halogenhaltigen Nitroverbindungen oder Dinitroverbindungen umgesetzt werden. Als aromatische Nitroverbindungen werden vorzugsweise 4-Chlornitrobenzol und 1,3-Dinitrobenzol eingesetzt. Die Umsetzung wird in einem organischen Lösemittel durchgeführt, und zwar in einem dipolaren aprotischen Lösemittel wie Dimethylformamid, Dimethylacetamid, Dimethylsulfoxid, Dimethylsulfon, Sulfolan, (Tetrahydrothiophen-1,1-dioxid), 1-Methyl-2-pyrrolidinon. Mindestens stöchiometrische Mengen einer basischen Verbindung, beispielsweise Alkalihydroxid, Ammoniumhydroxid, Alkalihydrid, Alkali- oder Erdalkalicarbonat oder -hydrogencarbonat sowie Alkalialkoholat, werden bei der Umsetzung eingesetzt. Die Molverhältnisse betragen 1 : 1 bis 4, vorzugsweise 1 : 1,1 bis 2, bezogen auf die Hydroxyphenyl-Verbindung. Die Reaktionstemperatur beträgt 50 bis 200 °C, vorzugsweise 100 bis 180 °C.

Die Reduktion der erhaltenen Nitroverbindungen wird nach üblichen katalytischen Methoden mit Hydrierkatalysatoren oder nach stöchiometrischen Methoden, beispielsweise mit Zinn(II)-chlorid-Eisessig, durchgeführt werden.

Als Katalysatoren für die katalytische Reduktion werden beispielsweise Platinmetalle, Kupfer, Eisen, Kobalt, Nickel, Gemische daraus oder Oxide der genannten Metalle bei atmosphärischem oder erhöhtem Druck eingesetzt. Palladium wird bevorzugt. Die Katalysatoren können als Metall selbst oder in feinverteilter Form auf Kohle, Bariumsulfat, Silikagel, Aluminium und Zeolith verwendet werden. Bekannt ist auch die Verwendung von Raney-Nickel. Die Reduktion findet in einem organischen Lösemittel, beispielsweise in einem Alkohol wie Methanol, Ethanol und Isopropylalkohol, Glykol wie Ethylenglykol und Propylenglykol, Ether wie Diethylether, Dioxan, Tetrahydrofuran und Ethylenglykolmonoethylether, aliphatischen Kohlenwasserstoff wie Hexan und Cyclohexan, aromatischen Kohlenwasserstoff wie Benzol, Toluol und Xylol, Ester wie Ethylacetat und Butylacetat, halogenierten Kohlenwasserstoff sowie Dimethylformamid und Dimethylsulfoxid statt. Die Temperatur bei dieser Reaktion liegt zwischen 10 und 130 °C, vorzugsweise zwischen 20 bis 80 °C.

Für die Herstellung der erfindungsgemäßen Mono- und Dicarbonsäurechloride sowie Mono- und Dicarbonsäureamide werden die allgemein üblichen Methoden zur Herstellung von Carbonsäurechloriden aus Carbonsäuren oder Carbonsäureamiden aus Carbonsäurechloriden angewendet.

Beispielsweise eignet sich die Umsetzung der Carbonsäuren mit Thionylchlorid, Phosphortrichlorid oder Phosphorpentachlorid, vorzugsweise mit Thionylchlorid, zur Herstellung der Carbonsäurechloride, welche anschließend mit wäßriger Ammoniak-Lösung in Dioxan zu den Carbonsäureamiden umgesetzt werden können.

Die Umlagerung der Carbonsäureamide zu den erfindungsgemäßen Aminen und Diaminen erfolgt unter den Bedingungen des Hofmannschen Carbonsäureamidabbaus. Bei der Hofmann-Reaktion werden Carbonsäureamide durch die Einwirkung von Hypohalogenit in Gegenwart von Basen in Amine umgewandelt. Als Hypohalogenit-Lösungen werden 5 bis 30gewichtsprozentige wäßrige Lösungen aus Alkalihypochloriten und -bromiten, vorzugsweise eine etwa 13gewichtsprozentige Natriumhypochlorit-Lösung, in Gegenwart von 5 bis 50 Gew.-% einer Base, beispielsweise eines Alkalihydroxids oder Ammoniumbase, eingesetzt. Das molare Verhältnis von Carbonsäureamid zu Hypohalogenit beträgt höchstens 1 : 5, vorzugsweise 1 : 1,25, wobei das Carbonsäureamid in einem organischen Lösemittel, vorzugsweise Ethanol oder Dioxan, suspendiert oder vollständig gelöst ist. Als organische Lösemittel können auch niedrige aliphatische Alkohole oder Ether, beispielsweise Diethylenglykoldimethylether, eingesetzt werden. Zur Reaktionsbeschleunigung können Phasentransferkatalysatoren, beispielsweise Tetraalkylammoniumsalze, Benzyltrialkylammoniumsalze, Benzyltrialkylphosphoniumsalze, Benzyltriphenylphosphoniumsalze, Tetraalkylphosphoniumsalze mit 1 bis 6 C-Atomen im Alkylrest, Kronenether oder Polyethylenglykole, in Mengen von 0,1 bis 20 Mol-% zugesetzt werden.

Die Reaktionstemperatur bei der Hofmann-Umlagerungsreaktion beträgt 10 bis 150 °C, vorzugsweise 70 bis 90 °C.

Für die Reinigung der Diamine werden diese bei 10 bis 100 °C mit Säuren in eines ihrer wasserlöslichen Salze (z. B. Halogenid, Hydrogensulfat) überführt, die gegenüber Aminogruppen unter den angewendeten Bedingungen inert sind.

Die erhaltenen Diaminoverbindungen eignen sich zur Herstellung von hochtemperaturbeständigen Polykondensaten, wie Polyamiden und Polyimiden.

Durch Umsetzung mit Tetracarbonsäure oder deren Derivaten werden Polyimide erhalten, die niedrige Dielektrizitätskonstanten aufweisen. Andererseits ergibt die Reaktion mit Dicarbonsäurechloriden Polyamide.

Weiterhin können neue Monomere und Oligomere durch Reaktion mit Dianhydriden erhalten werden. Die erhaltenen Imidmonomere und Oligomere können durch Additionsreaktionen gehärtet werden.

Die erfindungsgemäßen Diamine sind zur Herstellung von polymeren Vorprodukten, Epoxidharzhärtern, Matrixharzen, Laminaten, Filmen, Fasern, Klebstoffen, Beschichtungen, Fotoresists und Formkörpern geeignet.

Die Erfindung wird durch die Beispiele näher erläutert.

**Beispiel 1**

Herstellung von 2-(4-Hydroxyphenyl)-2-(4-methylphenyl)hexafluorpropan nach der Gleichung:

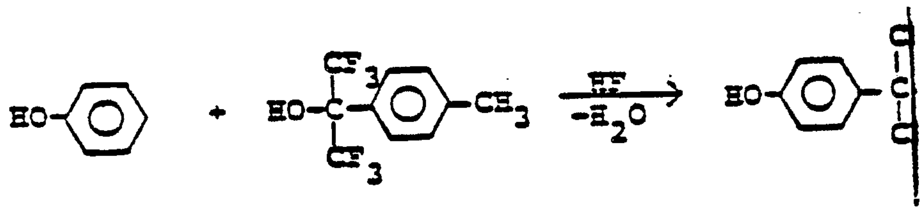


774 g (3 mol) 2-Hydroxy-2-(4-methylphenyl)hexafluorpropan, 282 g (3 mol) Phenol und 540 g (27 mol) wasserfreier Fluorwasserstoff wurden in einem 2-dm$^3$-Stahlautoklaven vereinigt und unter Rühren 60 h auf 110 °C erhitzt. Anschließend wurde der Fluorwasserstoff abgelassen und in $H_2O$ absorbiert. Der Autoklavenrückstand wurde nach Erkalten auf Eiswasser gegeben, die organische Phase mit 600 cm$^3$ $CH_2Cl_2$ verdünnt, zweimal mit Wasser gewaschen, über $CaCl_2$ getrocknet und wieder vom Lösemittel befreit. Dann wurde das Produkt destilliert.

Kp.$_{1,4}$: 154 °C, Ausbeute: 848 g ≙ 84,6 der Theorie.

Die Analyse des Produkts ergab folgendes Ergebnis:

| | | | | |
|---|---|---|---|---|
| $C_{16}H_{12}F_6O$ | ber.: | C 57,48 % | H 3,59 % | F 34,13 % |
| (MG: 334) | gef.: | C 56,70 % | H 3,50 % | F 33,80 %. |

**Beispiel 2**

Herstellung von 2-(4-Methylphenyl)-2-[4-(4-methylphenoxy)phenyl]hexafluorpropan nach der Gleichung:

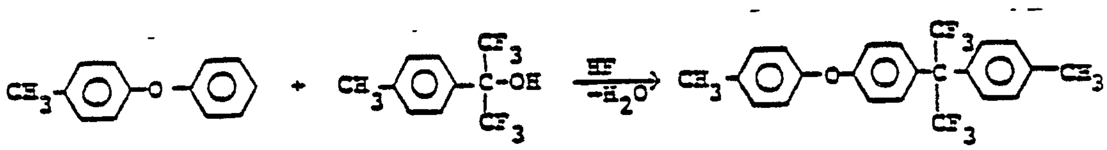


Ein Gemisch von 186 g (1,01 mol) 4-Methyldiphenylether, 258 g (1 mol) 2-Hydroxy-2-(4-methylphenyl)-hexafluorpropan und 180 g (9 mol) wasserfreier Fluorwasserstoff wurde in einem 1-dm$^3$-Stahlautoklaven 65 h bei 170 °C gerührt. Nach Abkühlen auf 80 °C wurde der Fluorwasserstoff abgelassen und in $H_2O$

absorbiert. Zu dem Rückstand wurden 200 cm³ $CH_2Cl_2$ gegeben. Die organische Phase wurde auf Eiswasser zur Entfernung restlicher Mengen HF gegossen, abgetrennt, zweimal mit Wasser gewaschen und über Calciumchlorid getrocknet. Anschließend wurde das Lösemittel am Rotationsverdampfer abdestilliert.

Der kristalline Rückstand (434 g) wurde in 450 cm³ Ethanol 20 g Aktivkohle aufgenommen und auf Siedetemperatur des Ethanols erhitzt. Nach Abtrennen der Aktivkohle ließ man die Lösung abkühlen. Das Kristallisat wog 207 g, Ausbeute:. 48,8 %. Fp.: 98,5 bis 99,5 °C.

Die Analyse des Produkts ergab folgendes Ergebnis:

| | | | | |
|---|---|---|---|---|
| $C_{23}H_{18}F_6O$ | ber.: | C 65,09 % | H 4,24 % | F 26,88 % |
| (MG: 424) | gef.: | C 64,80 % | H 4,30 % | F 26,60 %. |

**Beispiel 3**

Herstellung von 2-(4-Carboxyphenyl)-2-[4-(4-nitrophenoxy)phenyl]-hexafluorpropan nach der Gleichung:

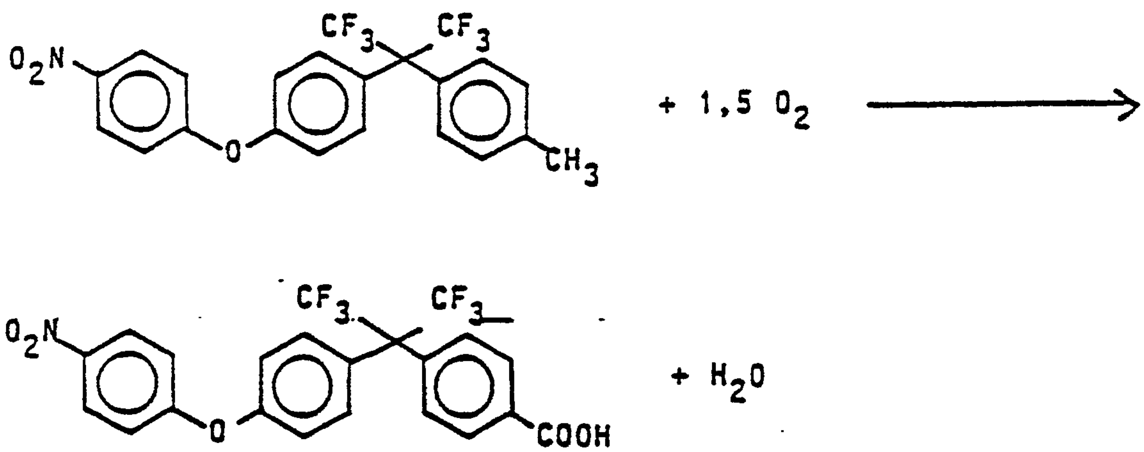

a) Oxidation

Eingesetzte Mengen:
240,0 g (0,527 mol) 2-(4-Methylphenyl)-2-[4-(4-nitrophenoxy)phenyl]hexafluorpropan
2,49 g (0,01 mol) $Co(OAc)_2 \cdot 4\ H_2O$
2,45 g (0,01 mol) $Mn(OAc)_2 \cdot 4\ H_2O$
0,41 g (0,005 mol) HBr entsprechen 4,1 g einer 10gewichtsprozentigen HBr-Lösung in Eisessig und
520 g Eisessig.

Die Mischung wurde unter 7,5 bar auf 185 °C erhitzt. Ab ca. 130 °C setzt die exotherme Reaktion ein, die ca. 25 Minuten anhielt. Anschließend wurde die Temperatur noch für 1 Stunde auf 173 bis 177 °C gehalten.

b) Aufarbeitung

Die Reaktionslösung (ca. 790 g) wurde heiß (100 °C) dem Autoklaven entnommen. Das Produkt war dann zum Teil schon auskristallisiert. Die Kristallsuspension wurde unter Rühren auf 22 °C abgekühlt, über eine Nutsche abgesaugt und viermal mit je 50 cm³ Eisessig gewaschen. Die gelben Kristalle wurden bei 80 °C/65 mbar im Luftstrom getrocknet.

Ausbeute: 232,5 g (90,9 % d. Th.) gelbe Kristalle.
Fp.: 205 bis 207 °C.
Fp. der Reinsubstanz: 208 °C.
Die Analyse des Produkts ergab folgendes Ergebnis:

| | | | | |
|---|---|---|---|---|
| $C_{22}H_{13}F_6NO_5$ | ber.: | C 54,44 % | H 2,70 % | N 2,89 % |
| (MG: 485,33) | gef.: | C 54,10 % | H 2,80 % | N 2,80 %. |

Durch Einengen der vereinigten Filtrate auf 130 g kristallisiert zusätzlich Carbonsäure aus.
Ausbeute: 4,35 g (1,7 % d. Th.) gelbe Kristalle.
Fp.: 198 bis 204,5 °C.

**Beispiel 4**

Herstellung von 2-(4-Carboxyphenyl)-2-[4-(3-nitrophenoxy)phenyl]hexafluorpropan mit der Formel

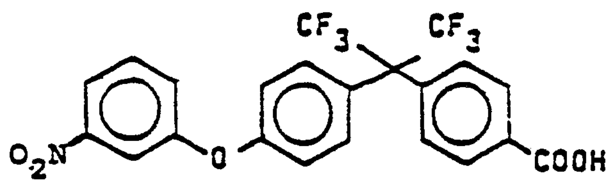

Es wurde analog zu Beispiel 3 gearbeitet.
Ausbeute: 85 % d. Th.
Fp.: 191 bis 193 °C.
Die Analyse des Produkts ergab folgendes Ergebnis:

| | | | | |
|---|---|---|---|---|
| $C_{22}H_{13}F_6NO_5$ | ber.: | C 54,44 % | H 2,70 % | N 2,89 % |
| (MG: 485,33) | gef.: | C 54,30 % | H 3,00 % | N 2,70 %. |

Beispiel 5

Herstellung von Hexafluor-2-(4-carboxyphenyl)-2-[4-(4-carboxyphenoxy)phenyl]propan nach der Gleichung:

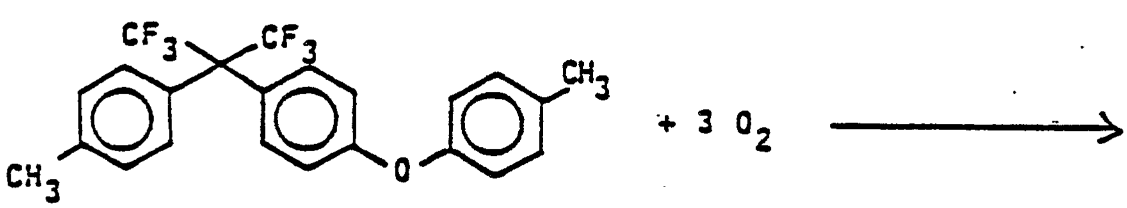

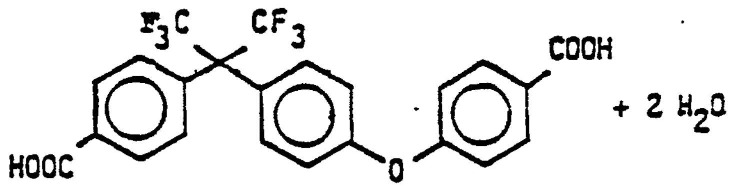

a) Reaktion

225 g (0,53 mol) Hexafluor-2-(4-methylphenyl)-2-[4-(4-methylphenoxy)phenyl]propan,
2,49 g (0,01 mol) $Co(OAc)_2 \cdot 4 H_2O$,
2,45 g (0,01 mol) $Mn(OAc)_2 \cdot 4 H_2O$,
0,41 g (0,005 mol) HBr entsprechen 4,1 g einer 10gewichtsprozentigen HBr-Lösung in Eisessig und
525 g Eisessig
wurden in einem 1-$dm^3$-Glasautoklav, der mit Rührer, Gaseinleitungsrohr, Thermometer und Rückflußkühler ausgerüstet war, vorgelegt.

Unter 6,5 bar Sauerstoffdruck wurde bis 175 °C erhitzt. Die exotherme Reaktion setzte unter Sauerstoffaufnahme bei ca. 110 °C ein und dauerte ca. 45 Minuten. Die Endtemperatur von 170 °C wurde noch 1 Stunde gehalten.

b) Aufarbeitung

Unterhalb 100 °C wurde dem Autoklaven eine Kristallsuspension entnommen, die nach weiterem Abkühlen auf 25 °C über eine Nutsche abgesaugt wurde. Der Filterkuchen wurde viermal mit je 50 $cm^3$ Eisessig und anschließend dreimal mit je 50 $cm^3$ Wasser gewaschen. Das feuchte Produkt wurde bei 80 °C/65 mbar im leichten Luftstrom getrocknet.
Ausbeute: 218,5 g (95,1 % d. Th.) farblose Kristalle.
Fp.: 268 bis 270 °C.
Eindampfen der Mutterlauge auf 1/7 des ursprünglichen Volumens ergab zusätzlich 16,5 g (6,4 % d. Th.) Dicarbonsäure.
Fp.: 261 bis 263 °C.

**Beispiel 6**

Herstellung von 2-(4-Chlorcarbonylphenyl)-2-[4-(4-chlorcarbonylphenoxy)phenyl]hexafluorpropan

121 g 2-(4-Carboxyphenyl)-2-[4-(4-carboxyphenoxy)phenyl]hexafluorpropan wurden mit 476 g Thionylchlorid und 0,5 $cm^3$ Dimethylformamid so lange unter Rückfluß erhitzt, bis keine Gasentwicklung mehr zu beobachten war. Das überschüssige Thionylchlorid wurde abdestilliert und das Rohprodukt aus Cyclohexan umkristallisiert.
Ausbeute: 122 g (94 % d. Th.).
Fp.: 90 bis 91 °C

Die Analyse des Produkts ergab folgendes Ergebnis:

| | | | | |
|---|---|---|---|---|
| $C_{23}H_{12}F_6Cl_2O_3$ | ber.: | C 52,99 % | H 2,32 % | Cl 13,60 % |
| (MG: 521,24) | gef.: | C 52,70 % | H 2,50 % | Cl 13,40 %. |

**Beispiel 7**

Herstellung von 2-(4-Carbonsäureamidophenyl)-2-[4-(4-carbonsäureamidophenoxy)phenyl]hexafluorpropan

In eine Lösung aus 62,5 g 2-(4-Chlorcarbonylphenyl)-2-[4-(4-chlorcarbonylphenoxy)phenyl]-hexafluorpropan wurden bei 5 bis 15 °C 11 g Ammoniak eingeleitet. Die Suspension wurde nach 1 Stunde in 3 $dm^3$ Wasser eingetragen und anschließend filtriert. Der abgetrennte Niederschlag wurde mit Wasser neutral gewaschen und nach dem Trocknen aus Methanol umkristallisiert.
Ausbeute: 49,2 g (85 % d. Th.), Fp.: 195 bis 196 °C.
Die Analyse des Produkts ergab folgendes Ergebnis:

| | | | | |
|---|---|---|---|---|
| $C_{23}H_{16}F_6N_2O_3$ | ber.: | C 57,26 % | H 3,34 % | N 5,81 % |
| (MG: 482,38) | gef.: | C 57,00 % | H 3,40 % | N 5,80 %. |

**Beispiel 8**

Herstellung von 4,4′-Bis[2-(4-carboxyphenyl)hexafluorisopropyl]diphenylether

250 g 4,4′-Bis[2-(4-methylphenyl)-hexafluorisopropyl] diphenylether, 2,49 g $Co(OAc)_2 \cdot 4\ H_2O$, 2,45 g $Mn(OAc)_2 \cdot 4\ H_2O$, 0,41 g HBr ≙ 4,1 g einer 10gewichtsprozentigen HBr-Lösung in Eisessig und 550 g Eisessig wurden im 1-$dm^3$-Glasautoklav, der mit Rührer, Gaseinleitungsrohr, Thermometer und Rückflußkühler ausgerüstet war, vorgelegt. Unter 7,5 bar Sauerstoffdruck wurde bis auf maximal 180 °C erhitzt. Die exotherme Reaktion setzte unter Sauerstoffaufnahme bei ca. 130 °C ein und dauerte 40 Minuten. Die Endtemperatur von 175 °C wurde noch eine Stunde gehalten. Aus der auf ca. 100 °C abgekühlten Reaktionslösung wurden 300 g Eisessig abdestilliert, und der Destillationsrückstand wurde unter Rühren auf 20 °C abgekühlt. Die gebildete Kristallsuspension wurde über eine Nutsche abgesaugt. Der Filterkuchen wurde viermal mit je 15 $cm^3$ Eisessig und anschließend fünfmal mit je 40 $cm^3$ Wasser gewaschen. Das feuchte Produkt wurde bei 70 °C/65 mbar im leichten Luftstrom getrocknet.
Ausbeute: 211,8 g (77,6 % d. Th.) farblose Kristalle.
Fp.: 238 bis 240 °C.
Carboxylgruppengehalt: 2,84 mVal COOH/g (ber. 2,82).
Aus der Mutterlauge fiel durch Zugabe des Waschwassers zusätzliches Produkt aus.
Ausbeute: 57,3 g (21,0 % d. Th.). Fp.: 227 bis 232 °C.
Die Analyse des Produkts ergab folgendes Ergebnis:

| | | | | |
|---|---|---|---|---|
| $C_{32}H_{18}F_{12}O_5$ | ber.: | C 54,08 % | H 2,53 % | F 32,11 % |
| | gef.: | C 54,00 % | H 2,60 % | F 32,00 %. |

Herstellung von 4,4′-Bis[2-(4-chlorcarbonylphenyl)hexafluorisopropyl]diphenylether

107 g 4,4′-Bis[2-(4-carboxyphenyl)hexafluorisopropyl]diphenylether wurden in 354 g Thionylchlorid suspendiert und nach der Zugabe von 0,5 $cm^3$ Dimethylformamid so lange unter Rückfluß erhitzt, bis keine Gasentwicklung mehr zu beobachten war. Das Thionylchlorid wurde abdestilliert und das Rohprodukt aus Acetonitril umkristallisiert. Ausbeute: 106 g (94,6 % d. Th.). Fp.: 147 bis 148 °C.
Die Analyse des Produkts ergab folgendes Ergebnis:

| | | | | |
|---|---|---|---|---|
| $C_{32}H_{16}F_{12}Cl_2O_3$ | ber.: | C 51,42 % | H 2,15 % | Cl 9,49 % |
| (MG: 747,36) | gef.: | C 51,00 % | H 2,20 % | Cl 9,50 %. |

**Beispiel 9**

Herstellung von 4,4'-Bis[2-(4-carbonsäureamidophenyl)hexafluorisopropyl]diphenylether

75 g 4,4'-Bis[2-(4-chlorcarbonylphenyl)hexafluorisopropyl]diphenylether wurden in 300 $cm^3$ Dioxan gelöst und bei 10 bis 20 °C tropfenweise mit 100 $cm^3$ konzentrierter Ammoniaklösung versetzt. Nach 1 Stunde wurde die Suspension mit halbkonzentrierter Salzsäure angesäuert (pH 4 bis 5), in 3 $dm^3$ Eiswasser eingetropft, der Feststoff abfiltriert und mit Wasser gewaschen. Das getrocknete Rohprodukt wurde aus Acetonitril umkristallisiert.
Ausbeute: 59 g (83 % d. Th.).

Die Analyse des Produkts ergab folgendes Ergebnis:

| | | | | |
|---|---|---|---|---|
| $C_{32}H_{20}F_{12}N_2O_3$ | ber.: | C 54,24 % | H 2,85 % | N 3,95 % |
| (MG: 708,50) | gef.: | C 54,30 % | H 2,80 % | N 4,10 %. |

**Beispiel 10**

Herstellung von 4,4'-Bis[2-(4-aminophenyl)hexafluorisopropyl]diphenylether

49,5 g 4,4'-Bis[2-(4-carbonsäureamidophenyl)hexafluorisopropyl]diphenylether wurden in 500 $cm^3$ Ethanol gelöst und bei 10 bis 20 °C mit 200 $cm^3$ 10gewichtsprozentigen Natronlauge und 100 g einer 13gewichtsprozentigen Natriumhypochlorit-Lösung versetzt. Die Reaktionsmischung wurde ca. 4 Stunden unter Rückfluß erhitzt und nach dem Erkalten filtriert. Der abgetrennte Feststoff wurde mit Ethanol gewaschen und anschließend verworfen. Die Mutterlauge wurde mit halbkonzentrierter Essigsäure neutralisiert und eingeengt. Der Rückstand wurde in 300 $cm^3$ Ether und 200 $cm^3$ Wasser aufgenommen, die organische Phase abgetrennt, über Magnesiumsulfat getrocknet und das Lösemittel abdestilliert. Das blaßgelbe Rohprodukt wurde in 500 $cm^3$ Ethanol gelöst und die Verunreinigungen durch Behandlung mit 2 g Aktivkohle entfernt. Nach dem Abtrennen des Ethanols verblieben 39 g (85 % d. Th.) eines weißen Feststoffs, dessen gaschromatographisch bestimmte Reinheit größer als 99,9 % war. Fp.: 125,5 bis 127 °C.

Die Analyse des Produkts ergab folgendes Ergebnis:

| | | | | |
|---|---|---|---|---|
| $C_{30}H_{20}F_{12}N_2O$ | ber.: | C 55,22 % | H 3,09 % | N 4,29 % |
| (MG: 652,48) | gef.: | C 55,40 % | H 3,20 % | N 4,20 %. |

**Beispiel 11**

Herstellung von 2-(4-Methylphenyl)-2-[4-(4-nitrophenoxy)phenyl]hexafluorpropan

334 g (1 mol) 2-(4-Hydroxyphenyl)-2-(4-methylphenyl)hexafluorpropan, 1,2 $dm^3$ Dimethylacetamid, 200 $cm^3$ Toluol und 44 g Natriumhydroxid wurden so lange unter Rückfluß erhitzt, bis kein Reaktionswasser mehr mit Hilfe eines Wasserabscheiders abgetrennt werden konnte. Hiernach wurde das Toluol abdestilliert, 164,4 g (1,1 mol) 4-Chlornitrobenzol zugesetzt und 48 Stunden unter Rückfluß erhitzt. Nach dem Abkühlen auf etwa 0 °C wurde der Feststoff abfiltriert, mit ca. 100 $cm^3$ Dimethylacetamid gewaschen und verworfen. Aus dem Filtrat wurde das Dimethylacetamid weitgehendst mittels einer Vakuumdestillation abdestilliert. Der ölige Rückstand wurde mit 800 $cm^3$ Methanol aufgenommen, wobei ein gelber Feststoff ausfiel. Der Feststoff wurde abgetrennt und getrocknet. Nach der Aufarbeitung der Mutterlauge wurden insgesamt 325 g (71 % d. Th.) eines gelben Feststoffs erhalten.

Fp.: 89 bis 90 °C.

Die Analyse des Produkts ergab folgendes Ergebnis:

| $C_{22}H_{15}F_6NO_3$ (MG: 455,35) | | | | | |
|---|---|---|---|---|---|
| ber.: | C 58,03 % | H 3,32 % | N 3,08 % | F 25,04 % | O 10,54 % |
| gef.: | C 58,30 % | H 3,20 % | N 3,20 % | F 25,40 % | O 10,50 %. |

**Beispiel 12**

Herstellung von 2-(4-Methylphenyl)-2-[4-(3-nitrophenoxy) phenyl]hexafluorpropan

334 g (1 mol) 2-(4-Hydroxyphenyl)-2-(4-methylphenyl)hexafluorpropan wurden in 1,5 $dm^3$ Dimethylformamid gelöst und nach der Zugabe von 276 g Kaliumcarbonat und 170 g (1,01 mol) m-Dinitrobenzol 24 Stunden unter Rückfluß erhitzt. Anschließend wurde das Dimethylformamid abdestilliert und der Rückstand aus Methanol umkristallisiert.

Ausbeute: 219 g (48 % d. Th.) brauner Feststoff.

Fp.: 65 - 67 °C.

Die Analyse des Produkts ergab folgendes Ergebnis:

| | | | | |
|---|---|---|---|---|
| $C_{22}H_{15}F_6NO_3$ (MG: 455,35) | ber.:<br>gef.: | C 58,03 %<br>C 57,70 % | H 3,32 %<br>H 3,20 % | N 3,08 %<br>N 3,20 % |

**Beispiel 13**

Herstellung von 2-(4-Chlorcarbonylphenyl)-2-[4-(3-nitrophenoxy)phenyl]hexafluorpropan

146 g (0,3 mol) 2-(4-Carboxyphenyl)-2-[4-(3-nitrophenoxy)phenyl]hexafluorpropan wurden in 714 g Thionylchlorid suspendiert und nach der Zugabe von 1 $cm^3$ Dimethylformamid so lange unter Rückfluß erhitzt, bis keine Gasentwicklung mehr zu beobachten war. Das Thionylchlorid wurde abdestilliert und der Rückstand aus Acetonitril umkristallisiert.

Ausbeute: 126 g (83 % d. Th.) hellbrauner Feststoff,

Fp.: 187 - 191 °C.

Die Analyse des Produkts ergab folgendes Ergebnis:

| | | | | |
|---|---|---|---|---|
| $C_{22}H_{12}F_6ClNO_4$ (MG: 503,78) | ber.:<br>gef.: | C 52,45 %<br>C 52,40 % | H 2,40 %<br>H 2,60 % | N 2,79 %<br>N 3,10 %. |

**Beispiel 14**

Herstellung von 2-(4-Chlorcarbonylphenyl)-2-[4-(4-nitro phenoxy)phenyl]hexafluorpropan

9,7 g (0,02 mol) 2-(4-Carboxyphenyl)-2-[4-(4-nitrophenoxy)phenyl]hexafluorpropan wurden in 47 $cm^3$

Thionylchlorid suspendiert und nach der Zugabe einiger Tropfen Dimethylformamid so lange unter Rückfluß erhitzt, bis keine Gasentwicklung mehr festzustellen war. Das Thionylchlorid wurde abdestilliert und der Rückstand aus Acetonitril umkristallisiert.
Ausbeute: 10 g (99 % d. Th.) blaßgelber Feststoff,
Fp.: 98 bis 101 °C.
Die Analyse des Produkts ergab folgendes Ergebnis:

| | | | | |
|---|---|---|---|---|
| $C_{22}H_{12}ClF_6NO_4$ | ber.: | C 52,45 % | H 2,40 % | N 2,79 % |
| (MG: 503,78) | gef.: | C 52,30 % | H 2,30 % | N 2,50 %. |

## Beispiel 15

Herstellung von 2-(4-Carbonsäureamidophenyl)-2-[4-(3-nitrophenoxy)phenyl]hexafluorpropan

11,1 g (0,022 mol) 2-(4-Chlorcarbonylphenyl)-2-[4-(3-nitrophenoxy)phenyl]hexafluorpropan wurden in 100 $cm^3$ Dioxan gelöst und bei 10 bis 15 °C mit 15 $cm^3$ konzentrierter Ammoniaklösung versetzt. Die Emulsion wurde 15 Minuten gerührt und in 800 $cm^3$ Wasser eingetropft. Der Feststoff wurde abfiltriert und mit Wasser neutral gewaschen. Der getrocknete Feststoff wurde aus Toluol umkristallisiert.
Ausbeute: 8 g (75 % d. Th.) hellbrauner Feststoff.
Fp.: 192 bis 194 °C.
Die Analyse des Produkts ergab folgendes Ergebnis:

| | | | | |
|---|---|---|---|---|
| $C_{22}H_{14}F_6N_2O_4$ | ber.: | C 54,55 % | H 2,91 % | N 5,79 % |
| (MG: 484,35) | gef.: | C 54,80 % | H 3,20 % | N 5,60 %. |

## Beispiel 16

Herstellung von 2-(4-Carbonsäureamidophenyl)-2-[4-(4-nitrophenoxy)phenyl]hexafluorpropan

180 g (0,357 mol) 2-(4-Chlorcarbonylphenyl)-2-[4-(4-nitrophenoxy)phenyl]hexafluorpropan wurden in 1 $dm^3$ Dioxan gelöst und bei 20 bis 30 °C tropfenweise mit 150 $cm^3$ konzentrierter Ammoniaklösung versetzt. Die Reaktionsmischung wurde 1 Stunde gerührt, mit Salzsäure (1 : 1) neutralisiert und anschließend in 4 $dm^3$ Wasser eingetragen. Der Niederschlag wurde abfiltriert und mit Wasser gewaschen.
Ausbeute: 170 g (98 % d. Th.) hellbrauner Feststoff.
Fp.: 208 bis 210 °C.
Die Analyse des Produkts ergab folgendes Ergebnis:

| | | | | |
|---|---|---|---|---|
| $C_{22}H_{14}F_6N_2O_4$ | ber.: | C 54,55 % | H 2,91 % | N 5,79 % |
| (MG: 484,35) | gef.: | C 54,70 % | H 2,70 % | N 5,80 %. |

## Beispiel 17

Herstellung von 2-(4-Aminophenyl)-2-[4-(4-nitrophenoxy)phenyl]hexafluorpropan

4,8 g (0,01 mol) 2-(4-Carbonsäureamidophenyl)-2-[4-(4-nitrophenoxy)phenyl]hexafluorpropan wurden in 150 $cm^3$ Ethanol und 40 $cm^3$ Dioxan gelöst. Die Lösung wurde mit 15 $cm^3$ 10gewichtsprozentiger Natriumhydroxidlösung und 7,5 g 13gewichtsprozentiger NaOCl-Lösung versetzt und 3 Stunden unter Rückfluß erhitzt. Nach dem Erhalten der Reaktionsmischung wurde der Feststoff abfiltriert, mit 20 $cm^3$ Ethanol gewaschen und verworfen. Das Filtrat wurde mit verdünnter Essigsäure neutralisiert und am Rotationsverdampfer eingeengt. Der Rückstand wurde in 50 $cm^3$ Wasser und 50 $cm^3$ Diethylether aufgenommen, die organische Phase abgetrennt und mit Wasser gewaschen. Die organische Phase wurde über Magnesiumsulfat getrocknet und vollständig eingeengt. Das Rohprodukt wurde aus Methanol umkristallisiert.
Ausbeute: 2,1 g (46 % d. Th.) gelber Feststoff.
Fp.: 119 bis 121 °C.

Die Analyse des Produkts ergab folgendes Ergebnis:

| | | | | |
|---|---|---|---|---|
| $C_{21}H_{14}F_6N_2O_3$ | ber.: | C 55,27 % | H 3,09 % | N 6,14 % |
| (MG: 456,34) | gef.: | C 55,50 % | H 2,90 % | N 6,30 %. |

**Beispiel 18**

Herstellung von 2-(4-Aminophenyl)-2-[4-(3-nitrophenoxy)phenyl]hexafluorpropan

48 g (0,1 mol) 2-(4-Carbonsäureamidophenyl)-2-[4-(3-nitrophenoxy)phenyl]hexafluorpropan wurden in 800 $cm^3$ Dioxan suspendiert und bei etwa 15 °C mit 200 $cm^3$ 10gewichtsprozentiger Natronlauge und 63 g 13gewichtsprozentiger NaOCl-Lösung versetzt. Die Reaktionsmischung wurde 3 Stunden unter Rückfluß erhitzt und nach dem Abkühlen auf 20 °C mit verdünnter Essigsäure neutralisiert. Die organische Phase wurde abgetrennt, die wäßrige Phase mit 200 $cm^3$ Ether extrahiert. Die organischen Phasen wurden vereinigt und am Rotationsverdampfer eingeengt. Der Rückstand wurde mit 300 $cm^3$ Ether und 300 $cm^3$ Wasser aufgenommen, die organische Phase abgetrennt und über Magnesiumsulfat getrocknet. Der nach dem Abtrennen des Ethers verbliebene Rückstand wurde aus Methanol umkristallisiert.
Ausbeute: 25,5 g (56 % d. Th.) gelber Feststoff.
Fp.: 79 bis 83 °C.

Die Analyse des Produkts ergab folgendes Ergebnis:

| | | | | |
|---|---|---|---|---|
| $C_{21}H_{14}F_6N_2O_3$ | ber.: | C 55,27 % | H 3,09 % | N 6,14 % |
| (MG: 456,34) | gef.: | C 55,10 % | H 2,70 % | N 6,30 %. |

**Beispiel 19**

Herstellung von 2-(4-Aminophenyl)-2-[4-(3-aminophenoxy)phenyl hexafluorpropan

22,8 g (0,05 mol) 2-(4-Aminophenyl)-2-[4-(3-nitrophenoxy)phenyl]hexafluorpropan wurden in 600 $cm^3$ Essigester gelöst und nach der Zugabe von 1 g Palladium auf Kohle in einem 1-$dm^3$-Stahlautoklaven bei etwa 25 °C mit Wasserstoff (100 bar) reduziert. Nach Beendigung der Wasserstoffaufnahme wurde der Katalysator abfiltriert und der Essigester vollständig abgetrennt. Das ölige, braune Rohprodukt wurde über basisches Aluminiumoxid mit Essigester als Elutionsmittel chromatographiert. Die Hauptfraktion wurde vollständig eingeengt, wonach 18 g eines hochviskosen, blaßgelben Rückstands erhalten wurden. 9,5 g des blaßgelben Rückstands wurden in 100 $cm^3$ Salzsäure (1 : 1) suspendiert und in der Siedehitze mit 400 $cm^3$ Wasser versetzt. Die trübe Lösung wurde in der Siedehitze zweimal mit je 5 g Aktivkohle behandelt. Die klare, farblose Lösung wurde auf 20 °C abgekühlt, wobei ein weißer Feststoff ausfiel. Die Suspension wurde unter Schutzgas (Stickstoff) mit verdünnter Ammoniaklösung auf einen pH-Wert von 8 - 9 eingestellt. Der

Feststoff wurde unter Schutzgas abfiltriert, mit Wasser gewaschen und getrocknet.
Ausbeute: 5,8 g (52 % d. Th.) grau-weißer Feststoff.
Fp.: 55 bis 59 °C (Reinheit laut GC: 99,1 %).

Die Analyse des Produkts ergab folgendes Ergebnis:

| $C_{21}H_{16}F_6N_2O$ (MG: 426,36) | | | | |
|---|---|---|---|---|
| ber.: | C 59,15 % | H 3,78 % | N 6,57 % | F 26,74 % |
| gef.: | C 59,00 % | H 3,50 % | N 6,60 % | F 26,60 %. |

**Beispiel 20**

Herstellung von 2-(4-Aminophenyl)-2-[4-(4-aminophenoxy)phenyl]hexafluorpropan

48 g (0,1 mol) 2-(4-Carbonsäureamidophenyl)-2-[4-(4-carbonsäureamidophenoxy)phenyl]-hexafluorpropan wurden in 2 $dm^3$ Ethanol gelöst und nach der Zugabe von 1 $cm^3$ (0,002 mol) Tricaprylyl-ammoniumchlorid bei 0 bis 5 °C mit 400 $cm^3$ 10gewichtsprozentiger Natronlauge und 168 g 13gewichtsprozentiger NaOCl-Lösung versetzt. Die Reaktionsmischung wurde 22 Stunden unter Rückfluß erhitzt und nach dem Abkühlen auf etwa 25 °C filtriert. Das Filtrat wurde im Wasserstrahlvakuum bis auf ca. 400 $cm^3$ eingeengt.

Der zweiphasige Rückstand wurde in 300 $cm^3$ Ether aufgenommen, die wäßrige Phase mit 200 $cm^3$ Ether extrahiert, die vereinigten organischen Phasen mit Wasser gewaschen und über Magnesiumsulfat getrocknet. Nach dem Abdestillieren des Ethers wurde der Rückstand in 100 $cm^3$ Salzsäure (1 : 1) suspendiert, die Suspension mit 1,5 $dm^3$ Wasser verdünnt und in der Siedehitze dreimal mit je 3 g Aktivkohle behandelt. Die klare Lösung wurde auf 25 °C abgekühlt, wobei ein weißer Feststoff ausfiel. Die Suspension wurde mit Stickstoff gesättigt und unter Stickstoff mit verdünnter Ammoniaklösung auf einen pH-Wert von 8 bis 9 eingestellt. Der Feststoff wurde unter Stickstoff über einer Umkehrfritte abfiltriert, mit Wasser gewaschen und getrocknet.
Ausbeute: 31 g (73 % d. Th.) weißer Feststoff.
Fp.: 85 bis 87 °C (Reinheit laut GC: 99,9 %).

Die Analyse des Produkts ergab folgendes Ergebnis:

| | | | | |
|---|---|---|---|---|
| $C_{21}H_{16}F_6N_2O$ | ber.: | C 59,15 % | H 3,78 % | N 6,57 % |
| (MG: 426,36) | gef.: | C 58,70 % | H 3,60 % | N 6,70 %. |

**Ansprüche**

1. Verbindungen der Formel I

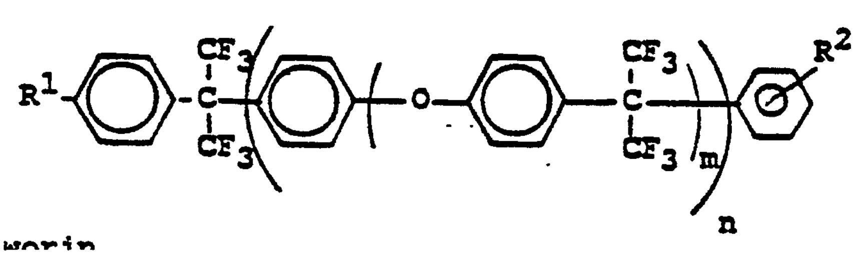

(I),

worin

m und n null oder 1 bedeuten, und wenn
m = null und n = null sind, dann sind
$R^1$ $-CH_3$ und
$R^2$ -OH in para-Stellung, wenn
m = null und n = 1 sind, dann sind
$R^1$ $-CH_3$, -COOH, -COCl, $-CONH_2$ und $-NH_2$ und
$R^2$ $-CH_3$, -COOH, -COCl, $-CONH_2$ $-NH_2$ und $-NO_2$ in meta-oder para-Stellung und wenn
m = 1 und n = 1 sind, dann sind
$R^1$ -COCl, $-CONH_2$, $-NH_2$ und
$R^2$ -COCl, $-CONH_2$ und $-NH_2$.

2. Verfahren zur Herstellung einer Verbindung der Formel I mit m = null und n = null gemäß Anspruch 1, dadurch gekennzeichnet, daß 2-(4-Methylphenyl)-2-hexafluorisopropanol mit Phenol in Gegenwart von wasserfreier Flußsäure zu 2-(4-Hydroxyphenyl)-2-(4-methylphenyl)hexafluorpropan umgesetzt wird.

3. Verfahren zur Herstellung von Verbindungen der Formel I mit m = null und n = 1 gemäß Anspruch 1, dadurch gekennzeichnet, daß

1) 2-(4-Hydroxyphenyl)-2-(4-methylphenyl)hexafluorpropan mit einer aromatischen halogenhaltigen Nitroverbindung oder Dinitroverbindung, oder 2-(4-Methylphenyl)-2-hexafluorisopropanol mit 4-Methyldiphenylether in Gegenwart von wasserfreier Flußsäure zu 2-(4-Methylphenyl)-2-[4-(4methylphenoxy)phenyl]-hexafluorpropan umgesetzt wird,

2) eine in 1) gebildete Verbindung zum Carbonsäurederivat oxidiert wird,

3) das Carbonsäurederivat mit Thionylchlorid zum Säurechlorid,

4) das Säurechlorid mit Ammoniaklösung zum Amid,

5) das Amid mit gelöstem Natriumhydroxid und NaOCl zum Amin umgesetzt und

6) die Nitrogruppe in der nach Ausführung von 5) vorliegenden Aminonitroverbindung katalytisch zum Amin reduziert wird.

4. Verfahren zur Herstellung von Verbindungen der Formel mit m = 1 und n = 1 gemäß Anspruch 1, dadurch gekennzeichnet, daß

1) 4,4′-Bis[2-(4-carboxyphenyl)hexafluorisopropyl]diphenylether mit Thionylchlorid zum Säurechlorid,

2) das Säurechlorid mit Ammoniaklösung zum Amid,

3) das Amid mit gelöstem Natriumhydroxid und NaOCl zum Amin umgesetzt werden.

5. Verfahren nach Anspruch 3, dadurch gekennzeichnet, daß die Oxidation einer in 1) gebildeten Verbindung durch Einleiten von Luftsauerstoff in ein saures organisches Medium bei einer Temperatur von 120 bis 220 °C und einem Druck zwischen 5 und 40 bar in Gegenwart eines Gemisches von Verbindungen der Metalle Kobalt und Mangan sowie von Bromidionen vorgenommen wird.

6. Verfahren nach Anspruch 3, dadurch gekennzeichnet, daß das Verhältnis von Kobalt zu Mangan gleich 3 : 1 bis 1 : 3 ist, wobei die Summe der Konzentrationen der beiden Elemente Kobalt und Mangan gleich 0,01 bis 0,20 g/Atom/kg Gesamtreaktionsmasse beträgt.

7. Verfahren nach Anspruch 3, dadurch gekennzeichnet, daß als zusätzliche Metallionen Cerionen im Katalysator im Verhältnis von Kobalt und Mangan zu Cer wie 1 : 0,02 bis 2 enthalten sind.

8. Verwendung der Verbindungen gemäß Anspruch 1 zur Herstellung von linearen Polycarbonsäureamiden und -imiden.

Europäisches Patentamt

# EUROPÄISCHER RECHERCHENBERICHT

Nummer der Anmeldung

EP 90 10 5404

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | EP-A-66298 (HITACHI)<br>* Seite 14 *<br>--- | 1, 8 | C07C39/367<br>C07C43/29<br>C07C205/38 |
| P,X | EP-A-317940 (HOECHST CELANESE)<br>* das ganze Dokument *<br>--- | 1-6, 8 | C07C65/24<br>C07C235/42<br>C07C217/80 |
| P,X | EP-A-317942 (HOECHST CELANESE)<br>* das ganze Dokument *<br>--- | 1-6, 8 | C07C41/09<br>C07C51/60<br>C07C201/12 |
| A | US-A-3355500 (BASIL S. FARAH ET AL.)<br>* Spalten 1 - 2 *<br>--- | 1 | C07C51/215<br>C07C213/02<br>C08G69/26 |
| A,P | EP-A-317883 (HOECHST AG.)<br>* das ganze Dokument *<br>----- | 1 | C07C217/82<br>C07C231/02 |

RECHERCHIERTE SACHGEBIETE (Int. Cl.5 )

C07C
C08G

Der vorliegende Recherchenbericht wurde für alle Patentansprüche erstellt

| Recherchenort | Abschlußdatum der Recherche | Prüfer |
|---|---|---|
| BERLIN | 03 JULI 1990 | RUFET, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument



EPO FORM 1503 03.82 (P0403)